# EUROPEAN PATENT SPECIFICATION

(11) **EP 1 013 291 B1**
(45) Date of publication and mention of the grant of the patent: **08.06.2005**
(21) Application number: 99125404.6
(22) Date of filing: 20.12.1999
(51) Int. Cl.: A61L 15/58, A61L 15/60

(54) **Hot melt adhesive comprising an absorbent**
Heizschmelzklebstoff ein Absorbens enthaltend
Adhesive thermofusible comprenant un absorbent

(30) Priority: 21.12.1998 US 217318
(43) Date of publication of application: 28.06.2000
(73) Proprietor: McNeil-PPC, Inc., Skillman, New Jersey 08558 (US)
(72) Inventor: Luizzi, Joseph, Newton, PA 18940 (US)
(74) Representative: Groening, Hans Wilhelm, Dipl.-Ing.

(56) References cited:
- EP-A- 0 658 351
- EP-A- 0 802 251
- WO-A-00/26293
- US-A- 5 372 870
- US-A- 5 503 919
- US-A- 5 750 623

## Description

### FIELD OF THE INVENTION

The present invention relates to a novel adhesive and more particularly to a hot-melt adhesive which is capable of absorbing liquids.

### BACKGROUND OF THE INVENTION

Absorbent articles such as catamenial pads, diapers, bandages, nursing pads and the like generally contain an absorbent element. The absorbent element of conventional disposable articles is typically formed from a fiberized wood pulp fluff or absorbent non-woven and/or other synthetic or natural absorbent materials such as peat moss or super-absorbent polymers. The absorbent element is covered with a soft, flexible liquid permeable topsheet which allows body fluid to be absorbed into the fluid retaining absorbent element. Typically a fluid impermeable backsheet is adhesively affixed to the liquid permeable topsheet around a peripheral edge margin to form a flange seal and thereby fully enclose the absorbent element to prevent fluid leakage.

Hot melt adhesives are typically used in the construction of absorbent articles to attach the liquid permeable topsheet to the absorbent element and also to attach the fluid impermeable backsheet to the element. In addition, hot melt adhesives are also used in the construction of the absorbent structures to laminate multiple plies together or to adhesively affix absorbent particles to a non-woven fabric or fibrous pulp.

EP 0 658 351 A1 discloses absorbent products and components for use in absorbent articles. These absorbent products comprise pressure-sensitive adhesive microfibers and thermoplastic polymer microfibers, that when incorporated into absorbent products provide good liquid transport properties, resiliency, and attachment systems. An absorbent powder may be immobilized on the pressure-sensitive adhesive microfiber coated surface of a substrate.

### SUMMARY OF THE INVENTION

It is an object of the present invention to provide a hot-melt adhesive which is capable of absorbing aqueous liquids.

It is another object of the present invention to provide a hot melt adhesive that eliminates the risk of super absorbent particles causing pinholes in a barrier backsheet when the absorbent article has been subjected to embossing or channeling.

It is another object of the present invention to provide a hot melt adhesive that eliminates the need to handle powders during the application of super absorbent particles to absorbent products

It is another object of the present invention to provide a hot melt adhesive which enables superabsorbent particles to be pattern coated onto a substrate.

It is another object of the present invention to provide a hot melt adhesive which also functions as a fluid retaining system.

. In accordance with the present invention, there has been provided a novel hot-melt adhesive that is capable of absorbing aqueous liquids which comprises in an uniform mixture:
about 10% to about 50% of a block copolymer the block copolymer being a linear or radial copolymer structure having the formula (A-B)ₓ wherein block A is a polyvinylarene block, block B is a poly(monoalkyl) block, x denotes the number of polymeric arms, and wherein x is an integer greater than or equal to one.
about 20% to about 80% of a tackifying resin; and
about 1% to about 60% of an aqueous liquid-absorbing polymer the aqueous liquid absorbing polymers being selected from the group consisting of thermoplastic hydrogels and thermoplastic polymeric compositions which are formed from a water-soluble soft segment and one ore more hard segments.

Also provided in accordance with the present invention is an absorbent article, the absorbent article comprising a liquid permeable topsheet, a liquid impermeable barrier sheet, an absorbent element between the topsheet and the barrier sheet, wherein either the topsheet or the barrier sheet is adhered to the absorbent element with a hot melt adhesive which further comprises in the
about 10% to about 50% of a block copolymer, the block copolymer being a linear or radial copolymer structure having the formula (A-B)ₓ wherein block A is a polyvinylarene block, block B is a poly(monoalkyl) block, x denotes the number of polymeric arms, and wherein x is an integer greater than or equal to one.
about 20% to about 80% of a tackifying resin; and
about 1% to about 60% of an aqueous liquid-absorbing polymer.

Also provided in accordance with the present invention is an absorbent article, the absorbent article comprising a liquid permeable topsheet, a liquid impermeable barrier sheet, an absorbent element between the topsheet and the barrier sheet, wherein at least a portion of the absorbent element contains a hot melt adhesive which further comprises in an uniform mixture:
about 10% to about 50% of a block copolymer, the block copolymer being a linear or radial copolymer structure having the formula (A-B)ₓ wherein block A is a polyvinylarene block, block B is a poly(monoalkyl) block, x denotes the number of polymeric arms, and wherein x is an integer greater than or equal to one.
about 20% to about 80% of a tackifying resin; and
about 1% to about 60% of an aqueous liquid-absorbing polymer.

### BRIEF DESCRIPTION OF THE DRAWINGS

Fig. **1** is a top plan view of a sanitary napkin having a pattern coating.
Figs. **2** is a side view of the sanitary napkin in Figure 1 taken through line A-A showing the adhesive applied between a cover layer and an absorbent element.
Fig. **3** is a top plan view of a sanitary napkin having a zone coating.
Fig. **4** is a top plan view of a sanitary napkin having a foamed adhesive in a pattern which forms side and end gaskets.
Fig. 5 is a side view of the sanitary napkin of Figure 4 taken through line B-B showing the adhesive applied on an upper surface of a cover layer of the napkin.
Fig. **6** is a top plan view of a sanitary napkin having a multi-line adhesive pattern.
Fig. **7** is a side view of the sanitary napkin of figure 6 taken through line C-C showing the adhesive applied between a barrier layer and an absorbent element.
Fig. **8** is a top plan view of a sanitary napkin having adhesive applied in a curved line pattern adjacent each longitudinal side edge and transverse end region of the napkin.
Fig. **9** is a top plan view of a sanitary napkin having adhesive applied in a curved line pattern which forms a closed perimeter around a center region of the sanitary napkin.
Fig. **10** is a top plan view of a sanitary napkin having adhesive applied in an hour glass pattern in a central region of the napkin.
Fig. **11** is a top plan view of a sanitary napkin having adhesive applied as a pair of substantially parallel lines adjacent each longitudinal side edge of the napkin.
Fig. **12** is a side view of the sanitary napkin of Figure 11 taken through lines D-D of Figure 11 showing the adhesive applied between a cover layer and an absorbent element of the napkin.

### DETAILED DESCRIPTION OF THE PREFERRED EMBODIMENTS

The present invention relates to hot melt adhesives, and more particularly to hot melt adhesives which are useful in the construction of absorbent articles such as catamenial pads, diapers, breast pads, surgical pads and bandages. Unlike traditional hot melt adhesives that are hydrophobic in nature, the hot-melt adhesives of the present invention readily absorb aqueous fluids such as saline and menstrual fluid. The hot melt adhesives of the present invention are formed from a blend of about 10% to about 50% of a block copolymer; about 20% to about 80% of a tackifying resin; and about 1% to about 60% of an aqueous liquid-absorbing polymer. The hot melt adhesives of the present invention may optionally contain absorbent thermoplastic polymers, super absorbent particles, tackifiers and plasticizers.

In a preferred embodiment, the hot melt adhesives of the present invention comprise (by weight):
about 10 - 50% block copolymer;
about 20 - 80% tackifying resin;
about 1 - 60% aqueous liquid absorbing polymer;
about 0 - 40% plasticizer; and
about 0 - 2.0% antioxidant.

Block copolymers for use in the invention are linear or radial co-polymer structures having the formula (A-B)ₓ wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton™ elastomers from Shell Chemical Company, vector™ elastomers from Dexco, Solprene™ from Enichem Elastomers and Stereon™ from Firestone Tire & Rubber Co.

Suitable tackifying resins include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof. Commercial examples of these types of resins include Foral® hydrogenated rosin ester, Staybelite® hydrogenated modified rosin, Poly-pale® polymerized rosin, Permalyn® rosin ester, Pentalyn® rosin ester, Adtac® oil extended hydrocarbon resin, Piccopale® aromatic hydrocarbon, Piccotac®. Hercotac® aromatic modified aliphatic hydrocarbon, Regalrez® cycloaliphatic resins, or Piccolyte® from Hercules, Eselementz® from Exxon Chemical aliphatic hydrocarbon and cycloaliphatic resins, Wingtack® from Goodyear Tire & Rubber Co. synthetic polyterpene resins including aromatic modified versions, Arkon® partially and fully hydrogenated aromatic resins from Arakawa Chemicals, Zonatac® styrenated terpene resin, Zonarez® rosin ester and Zonester® rosin ester from Arizona Chemical and Nevtac® aromatic modified aliphatic hydrocarbon from Neville Chemical Company.

Aqueous liquid absorbing polymers are thermoplastic hydrogels such as superabsorbent materials or thermoplastic polymeric compositions, which are formed from a water-soluble soft segment and one or more hard segments. The hard segment must melt processable, i.e. at use temperature the hard segments in the polymer are below their melt temperature, and at process temperature, the hard segments are above their melting point temperature and below the decomposition temperature of either the other components of the hot-melt adhesive composition. The hard segment is substantially insoluble in water, and phase separates from the soft segment. Examples of suitable hard segments include, but are not limited to polyurethane, polyamides, polyesters, polyureas, and combinations thereof. Examples of suitable soft segments include, but are not limited to polyethylene oxide, polyvinyl alcohol, polyvinyl pyrrolidone, polyacrylamide, polysaccharide, polymaleic anhydride, random copolymers of polyethylene oxide and polypropylene-oxide and combinations thereof. The soft and hard segments may be covalently bonded together by means of urethane, amide, ester, or secondary urea linkages or combinations thereof. Examples of aqueous liquid absorbing thermoplastic polymeric compositions which are commercially available include hydrophilic polyurethane from Tyndale Plains-Hunter Ltd. and Aquacaulk® thermoplastic polymers from Sumitomo Seika Chemicals Co., Ltd.

Suitable superabsorbent materials include any of the conventional superabsorbent particles or superabsorbent fibers which are commercially available today. The superabsorbent material is preferably a superabsorbent particle having an average particle size less than 150 microns. An example of which is Aquakeep® J-550P from Absorbent Technologies Inc.

Suitable plasticizers for use in the present invention generally will include any conventional plasticizers which decrease hardness and modulus, enhance pressure sensitive tack and reduce melt and solution viscosity. It is preferred that the plasticizer be water soluble or water dispersible or alternatively be a wax-like substance such as polyethylene glycol, glycerin, glycerol, polypropylene glycol, butylene glycol or sorbitol. An example of a preferred plastizer is Carbowax® polyethylene glycol from Union Carbide.

Suitable anti-oxidants for use in the present invention include any conventional anti-oxidants, and are preferably hindered phenols such as for example Ethanox 330™ 1,3,5-trimethyl-2,4,6-tris(3,5-di-tert-butyl-4-hydroxybenzyl) benzene which is commercially available from the Ethyl Corporation.

The hot melt adhesives of the present invention may be formed by blending the block copolymer, the tackifying resin and the aqueous liquid-absorbing polymer in a suitable adhesive processing equipment such as a melt mixer or extruder at a temperature above their respective melting points until uniformly mixec. The hot melt adhesive may be applied to substrates using conventional adhesive application equipment such as a hot melt adhesive slot coating head, a hot melt adhesive swirl spray applicator (a commercial example of which is a Nordson Control Fiberization®), using a hot melt adhesive micro fiber applicator (commercial examples of these applicators include Nordson Control Coat®, ITW Dynafiber®, J&M Meltblown, and May Coating's Accufiber®), using a hot melt adhesive rotary screen applicator to create a pattern coating (examples of this equipment include Nordson and Kraemer rotary screen technology).

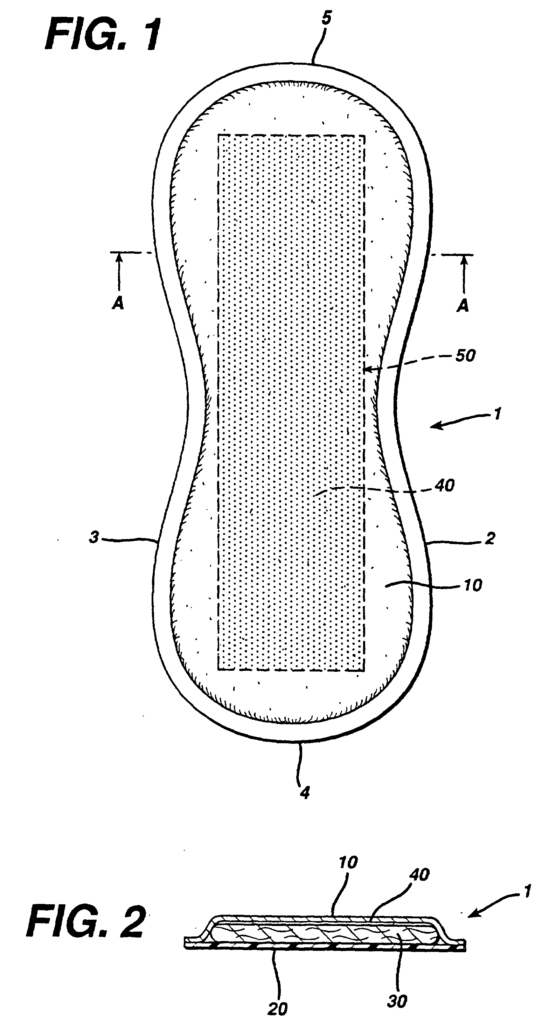

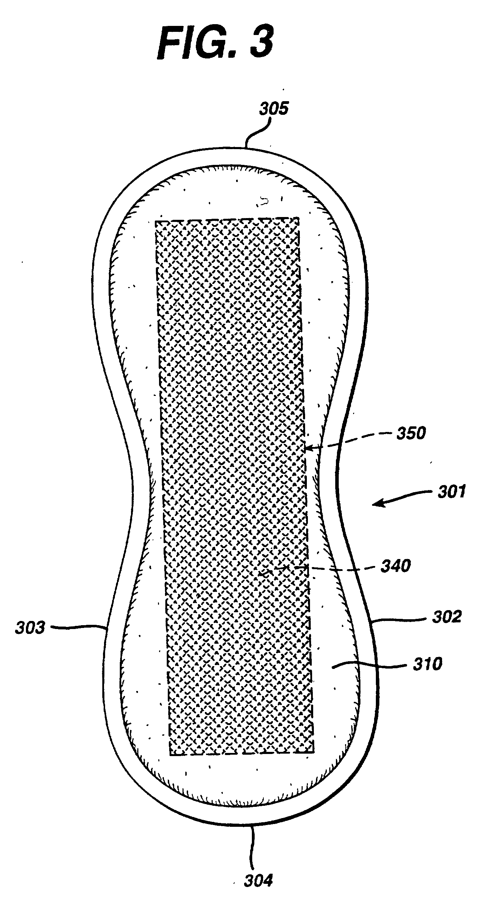

Referring to Figure 1, there is shown an absorbent article, which for purposes of illustration is a sanitary napkin **1** having opposite longitudinal sides **2**, **3** and opposite transverse ends **4**, **5**. Referring to Figure 2, the sanitary napkin **1** of Figure 1 is shown in cross section, having an upper, body facing, cover layer **10**, a lower garment facing, barrier layer **20** and absorbent element **30** between the cover layer **10** and barrier layer **20**. Liquid absorbing hot melt adhesive **40** adheres the cover layer **10** to the absorbent element **30** in a pattern coated absorbency zone **50** in a substantially rectangular pattern. The hot melt adhesive may alternatively be located between the absorbent element **30** and the barrier layer **20** (not shown). Other adhesive patterns and application locations are illustrated in Figures 3 to 12.

For example, Figure 3, shows a top plan view of an absorbent article **301** having cover layer **310**, opposite longitudinal sides **302**, **303** in an hour-glass configuration and opposite transverse ends **304**, **305**. Liquid absorbing hot melt adhesive **40** is zone coated in absorbency zone **350** in a substantially rectangular pattern.

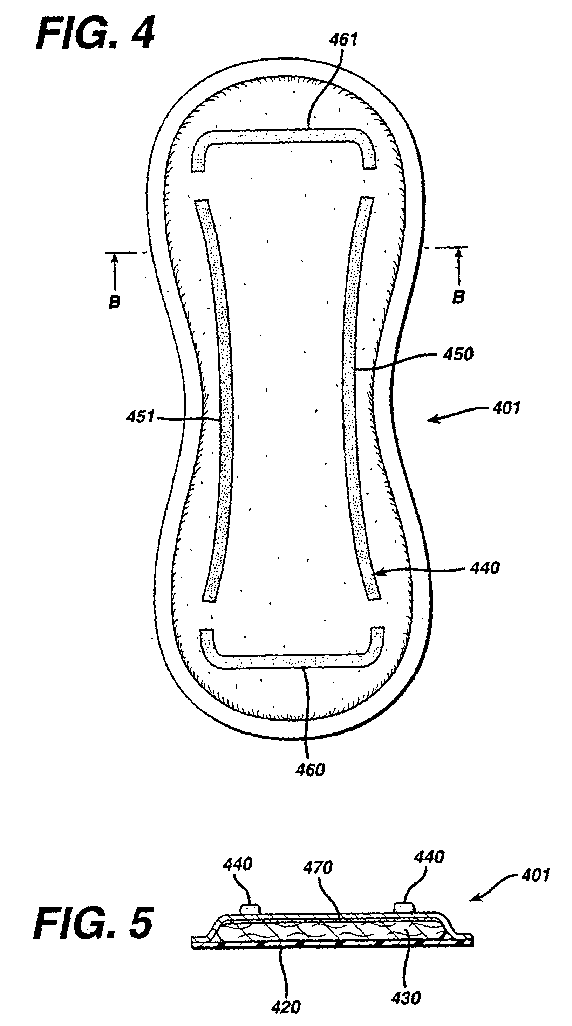

In the embodiment of the invention illustrated in Figures 4 and 5, there is shown sanitary napkin **401** having cover layer **401**, barrier layer **410**, absorbent element **430**, transfer layer **470**, and aqueous liquid absorbing hot melt adhesive **440** which has been foamed by mixing the adhesive with an inert gas. The foamed adhesive is then metered and dispensed through a nozzle onto a substrate, in this case the cover layer of a sanitary napkin. This technology is commercially available from the Nordson Corporation using their FoamMelt® processors and is disclosed more fully in Dilnik et al., U.S. Patent No. 5,807,367, . As shown in Figure 4, the foamed hot melt adhesive **440** has been applied to the body facing cover layer adjacent the longitudinal sides of the sanitary napkin **401** to form side gaskets **450**, **451** and end gaskets **460**,**461**. The hot melt adhesive **440** may alternatively be applied between the cover layer **410** and the subjacent absorbent element **430** (not shown).

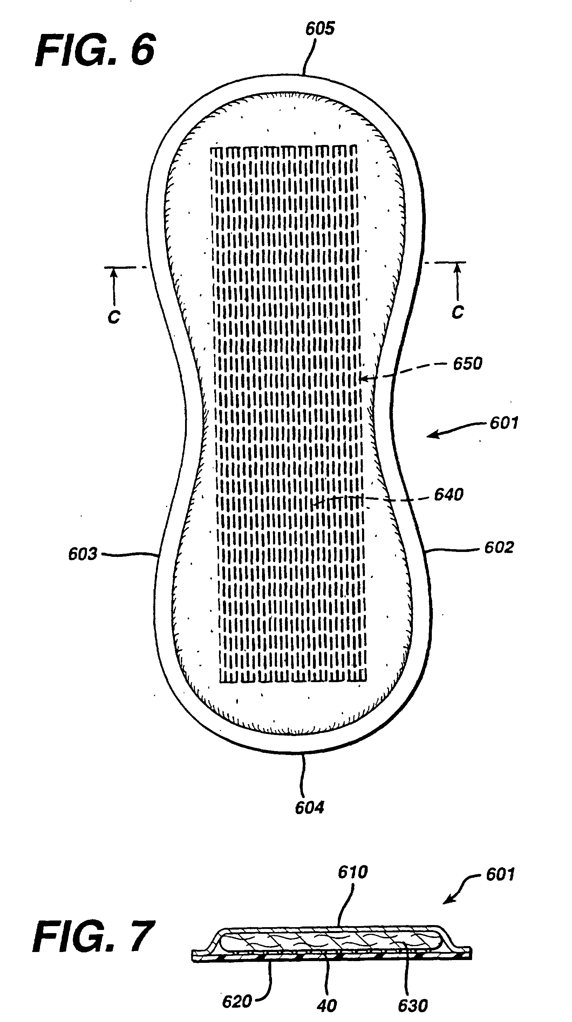

Referring to Figures 6 and 7, there is shown an absorbent article, which for purposes of illustration is a sanitary napkin **601** having opposite longitudinal sides **602**, **603** and opposite transverse ends **604**, **605**. Referring to Figure 7, the sanitary napkin **601** of Figure 6 is shown in cross section, having an upper, body facing, cover layer **610**, a lower garment facing, barrier layer **620** and absorbent element **630** between the cover layer **610** and barrier layer **620**. Liquid absorbing hot melt adhesive **640** adheres the barrier layer **620** to the absorbent element **630** in a multi-line coated absorbency zone **650** in a substantially rectangular pattern.

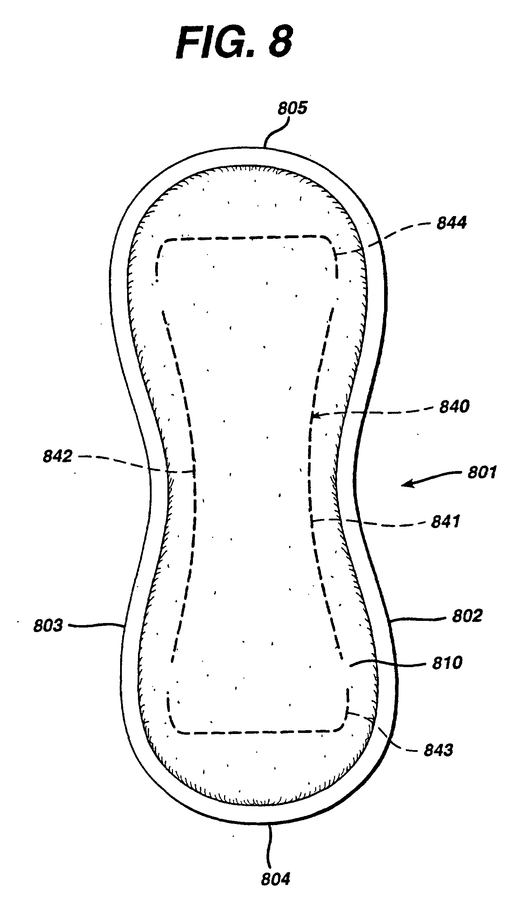

Figure 8 shows a top plan view of an absorbent article **801** having cover layer **810**, opposite longitudinal sides **802**, **803** in an hour-glass configuration and opposite transverse ends **804**, **805**. Liquid absorbing hot melt adhesive **840** is applied in a curved line pattern to form opposite side absorbency zones **841**, **842** and opposite transverse end absorbency zones **843**, **844**.

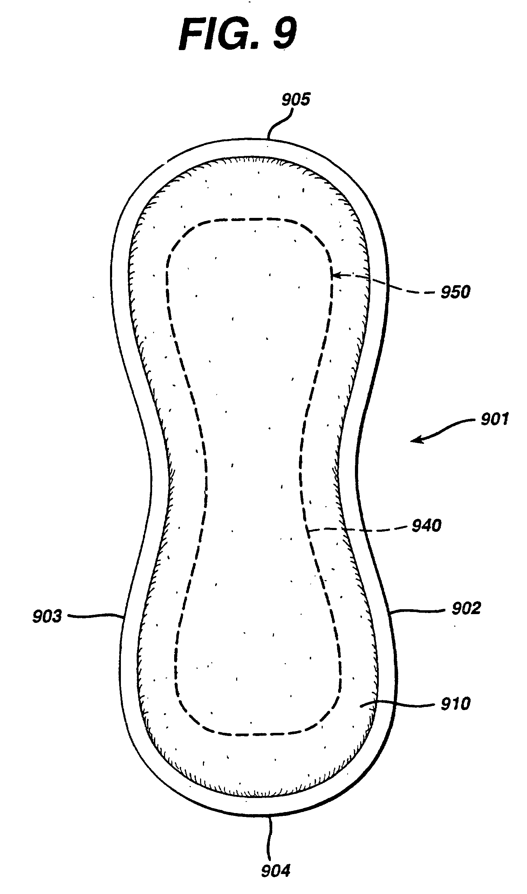

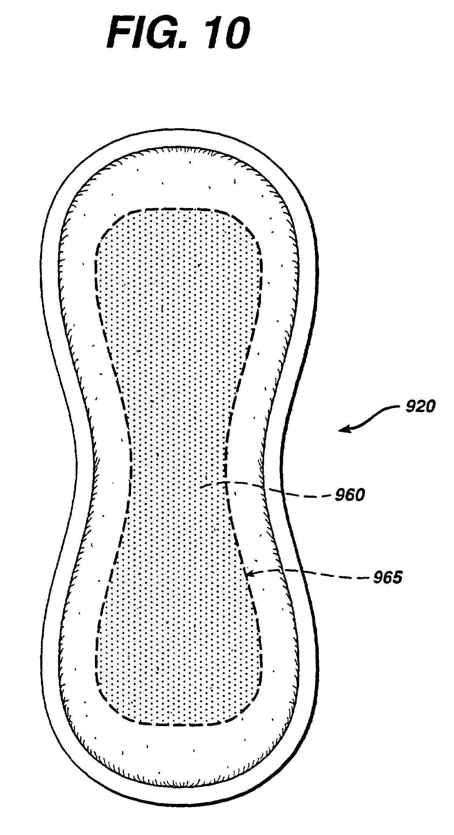

Figures 9 and 10 show top plan view of absorbent articles **901**, **920**, respectively, having an adhesive pattern in a substantially hour-glass shape. Figure 9 has cover layer **910**, opposite longitudinal sides **902**, **903** in an hour-glass configuration and opposite transverse ends **904**, **905**. Referring again to Figures 9 and 10, liquid absorbing hot melt adhesive **940**, **960** is applied to the article to form an hour-glass shaped absorbency zone **950**, **965** (respectively).

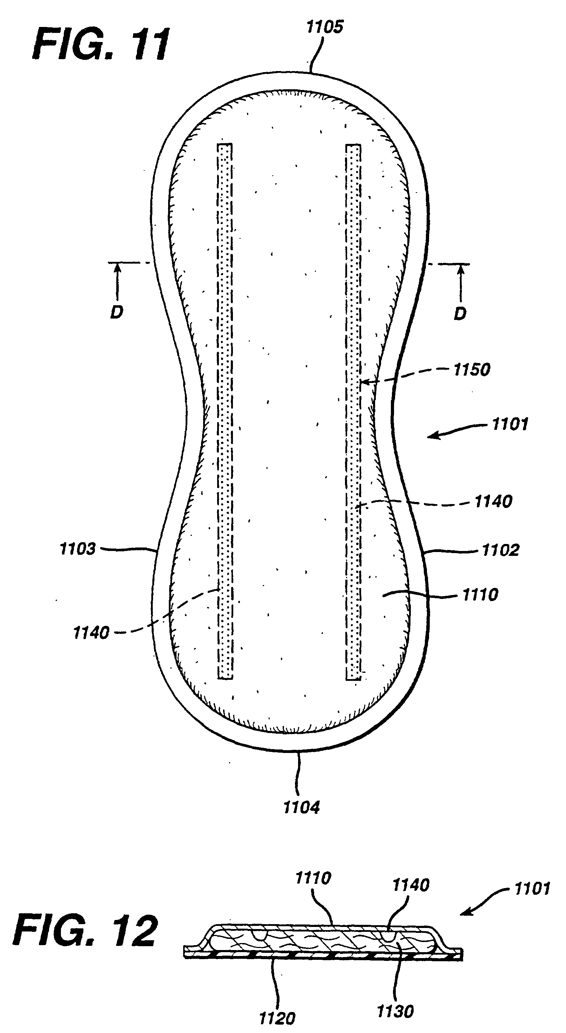

Referring to Figures 11 and 12, there is shown an absorbent article, which for purposes of illustration is a sanitary napkin **1101** having opposite longitudinal sides **1102**, **1103** and opposite transverse ends **1104**, **1105**. Referring to Figure 12, the sanitary napkin **1101** of Figure 11 is shown in cross section, having an upper, body facing, cover layer **1110**, a lower garment facing, barrier layer **1120** and absorbent element **1130** between the cover layer **1110** and barrier layer **1120**. Liquid absorbing hot melt adhesive **1140** is adhered to the cover layer **1110** and to the absorbent element **1130** in a parallel line coated absorbency zone **1150**.

### Example 1

The aqueous liquid absorbing hot-melt adhesive of the present invention was evaluated for its melt viscosity, adhesive strength (peel strength as measured on a polypropylene nonwoven fabric) and ability to absorb an aqueous saline solution (absorbent capacity) relative to a conventional hot melt adhesive. The conventional hot-melt adhesive was commercially available from the Fuller Company under the tradename HL-1491™. This is a standard hot-melt adhesive that is often used in the construction of absorbent articles such as sanitary napkins, panty liners, diapers and the like. The formulation of the conventional adhesive is believed to be in the following approximate proportions:
15-20% of a styrene-isoprene-styrene block copolymer having a 30% styrene content;
60 - 70% aliphatic or aromatic modified aliphatic tackifying resin
15-20% mineral oil
< 2% anti-oxidant
< 2% additional adjuncts (wax and polyethylene)

Two examples of the hot-melt adhesive of the present invention had the following formulas:
Sample A
   5.7% block copolymer (Vector DPX-552™)
   33.7% tackifying resin (Foral 85™)
   10.0% aq. liquid absorbing polymer (Aquacaulk TQU-5™)
   40.0% superabsorbent particles (Aquakeep J55-P™)
   10.5% plasticizer (Peg 600™) and
   0.5% antioxidant (Ethanox 330™).
Sample B
   15.0% block copolymer (Vector DPX-552™)
   25.0% tackifying resin (Foral 85™)
   0.0% aq. liquid absorbing polymer (Aquacaulk TQU-5™)
   45.0% superabsorbent particles (Aquakeep J55-P™)
   15.0% plasticizer (Peg 600™) and
   0.5% antioxidant (Ethanox 330™).

The results of the evaluation are provided in the following Table 1.

**Table 1**

| | Commercial Sample | Sample A | Sample B |
|---|---|---|---|
| Viscosity @ 177°C, 101,000 (Centipoise) | 1100 | 10,000 | |
| Absorbent Capacity (g/g) | none | 9.4 | 10.3 |
| Peel Strength (lbs./in. width) | 1.1 | 0.3 | 1.18 |

The commercially available hot-melt adhesive exhibited substantially no absorbency while the hot-melt adhesive of the present invention absorbed about 10 grams of liquid per gram of adhesive. Moreover, the adhesive composition of the present invention experienced no diminution of its adhesive strength.

## Claims

1. A hot-melt adhesive that is capable of absorbing aqueous liquids which comprises in an uniform mixture:
about 10% to about 50% of a block copolymer, the block copolymer being a linear or radial copolymer structure having the formula (A-B)ₓ wherein block A is a polyvinylarene block, block B is a poly(monoalkenyl) block, x denotes the number of polymeric arms, and wherein x is an integer greater than or equal to one;
about 20% to about 80% of a tackifying resin;
and about 1% to about 60% of an aqueous liquid-absorbing polymer, wherein the aqueous liquid absorbing polymers is selected from the group consisting of thermoplastic hydrogels and thermoplastic polymeric compositions which are formed from a water-soluble soft segment and one or more hard segments.

2. The hot-melt adhesive according to claim 1 wherein the block A polyvinylarenes is selected from the group consisting of Polystyrene, Polyalpha-methylstyrene, Polyvinyltoluene, and combinations thereof and wherein the Block B poly(monoalkenyl) blocks are selected from the group consisting of conjugated diene elastomers, hydrogenated elastomers and combinations thereof

3. The hot-melt adhesive according to claim 2 wherein the conjugated diene elastomers are selected from the group consisting of polybutadiene and polyisoprene and wherein the hydrogenated elastomers are selected from the group consisting of ethylene butylene, ethylene propylene, polyisobutylene and combinations thereof

4. The hot-melt adhesive according to claim 1 wherein the hot melt adhesive further contains absorbent thermoplastic polymers, super absorbent particles, tackifiers and plasticizers.

5. The hot-melt adhesive according to claim 1 wherein the tackifying resins are selected from the group consisting of natural resins, modified resins, glycerol esters of natural resins, glycerol esters of modified resins, pentaerythritol esters of natural resins, pentaerythritol esters of modified resins; polyterpene resins, copolymers of natural terpenes, terpolymers of natural terpenes, phenolic modified terpene resins and hydrogenated derivatives thereof, aliphatic petroleum resins and hydrogenated derivatives thereof, aromatic petroleum resin and hydrogenated derivatives thereof, aliphatic petroleum resins, hydrogenated derivatives of aliphatic petroleum resins, aromatic petrolium resins, hydrogenated derivatives of aromatic petroleum resins, and combinations thereof

6. The hot-melt adhesive according to any of the preceding claims wherein the hard segments are selected from the group consisting of polyurethane, polyamides, polyesters, polyureas, polypropylene oxide and combinations thereof.

7. The hot-melt adhesive according to any of the preceding claims, wherein the soft segments are selected from the group consisting of polyethylene oxide, polyvinyl alcohol, polyvinyl pyrrolidone, polyacrylamide, polysaccharide, polymaleic anhydride, and random copolymers of polyethylene oxide and polypropylene-oxide.

8. An absorbent absorbent article comprising a liquid permeable topsheet, a liquid impermeable barrier sheet, an absorbent element between the topsheet and the barrier sheet, wherein either the topsheet or the barrier sheet is adhered to the absorbent element with a hot melt adhesive which further comprises in an uniform mixture:
about 10% to about 50 % of a block copolymer, the block copolymer being a linear or radial copolymer structure having the formula (A-B)ₓ wherein block A is a polyvinylarene block, block B is a poly(monoalkenyl) block, x denotes the number of polymeric arms, and wherein x is an integer greater than or equal to one;
about 20% to about 80% of a tackifying resin; and
about 1% to about 60% of an aqueous liquid-absorbing polymer.

9. An absorbent article comprising a liquid permeable topsheet, a liquid impermeable barrier sheet, an absorbent element between the topsheet and the barrier sheet, wherein at least a portion of the absorbent element contains a hot melt adhesive which further comprises in an uniform mixture:
about 10% to about 50% of a block copolymer, the block copolymer being a linear or radial copolymer structure having the formula (A-B)ₓ wherein block A is a polyvinylarene block, block B is a poly(monoalkenyl) block, x denotes the number of polymeric arms, and wherein x is an integer greater than or equal to one;
about 20% to about 80% of a tackifying resin; and
about 1% to about 60% of an aqueous liquid-absorbing polymer.

## Patentansprüche

1. Heißschmelzklebstoff, der in der Lage ist, wäßrige Flüssigkeiten zu absorbieren, welcher in einer einheitlichen Mischung umfaßt:
etwa 10% bis etwa 50% eines Blockcopolymers, wobei das Blockcopolymer eine lineare oder radiale Copolymerstruktur mit der Formel (A-B)ₓ ist, wobei Block A ein Polyvinylarenblock ist, Block B ein Poly(monoalkenyl)block ist, x die Anzahl an polymeren Armen bezeichnet und wobei x eine ganze Zahl größer oder gleich Eins ist;
etwa 20% bis etwa 80% eines klebrig machenden Harzes;
und etwa 1 % bis etwa 60% eines wäßrigen, Flüssigkeit absorbierenden Polymers, wobei das wäßrige, Flüssigkeit absorbierende Polymer ausgewählt ist aus der Gruppe bestehend aus thermoplastischen Hydrogelen und thermoplastischen polymeren Zusammensetzungen, welche gebildet sind aus einem wasserlöslichen, weichen Segment und einem oder mehreren harten Segmenten.

2. Heißschmelzklebstoff nach Anspruch 1, **dadurch gekennzeichnet, daß** das Poylvinylaren von Block A ausgewählt ist aus der Gruppe bestehend aus Polystyrol, Polyalphamethylstyrol, Polyvinyltoluol und Kombinationen derselben, und wobei die Poly(monoalkenyl)blöcke von Block B ausgewählt sind aus der Gruppe bestehend aus konjugierten Dienelastomeren, hydrierten Elastomeren und Kombinationen derselben.

3. Heißschmelzklebstoff nach Anspruch 2, **dadurch gekennzeichnet, daß** die konjugierten Dienelastomere ausgewählt sind aus der Gruppe bestehend aus Polybutadien und Polyisopren, und wobei die hydrierten Elastomere ausgewählt sind aus der Gruppe bestehend aus Ethylenbutylen, Ethylenpropylen, Polyisobutylen und Kombinationen derselben.

4. Heißschmelzklebstoff nach Anspruch 1, **dadurch gekennzeichnet, daß** der Heißschmelzklebstoff ferner thermoplastische Absorbenspolymere, Superabsorbenspartikel, Klebrigmacher und Weichmacher enthält.

5. Heißschmelzklebstoff nach Anspruch 1, **dadurch gekennzeichnet, daß** die klebrig machenden Harze ausgewählt sind aus der Gruppe bestehend aus natürlichen Harzen, modifizierten Harzen, Glycerolestern von natürlichen Harzen, Glycerolestern von modifizierten Harzen, Pentaerythritolestern von natürlichen Harzen, Pentaerythritolestern von modifizierten Harzen; Polyterpenharzen, Copolymeren von natürlichen Terpenen, Terpolymeren von natürlichen Terpenen, phenolisch modifizierten Terpenharzen und hydrierten Derivaten derselben, aliphatischen Erdölharzen und hydrierten Derivaten derselben, aromatischen Erdölharzen und hydrierten Derivaten derselben, aliphatischen Petroleumharzen, hydrierten Derivaten von aliphatischen Petroleumharzen, aromatischen Petroleumharzen, hydrierten Derivaten von aromatischen Petroleumharzen und Kombinationen derselben.

6. Heißschmelzklebstoff nach einem der vorangehenden Ansprüche, **dadurch gekennzeichnet, daß** die harten Segmente ausgewählt sind aus der Gruppe bestehend aus Polyurethan, Polyamiden, Polyestern, Polyharnstoffen, Poylpropylenoxid und Kombinationen derselben.

7. Heißschmelzklebstoff nach einem der vorangehenden Ansprüche, **dadurch gekennzeichnet, daß** die weichen Segmente ausgewählt sind aus der Gruppe bestehend aus Polyethylenoxid, Polyvinylalkohol, Polyvinylpyrrolidon, Polyacrylamid, Polysaccharid, Polymaleinsäureanhydrid und statistischen Copolymeren von Polyethylenoxid und Poylpropylenoxid.

8. Absorbensgegenstand, der eine flüssigkeitspermeable Oberlage, eine flüssigkeitsimpermeable Barrierelage, ein Absorbenselement zwischen der Oberlage und der Barrierelage umfaßt, wobei entweder die Oberlage oder die Barrierelage an dem Absorbenselement mit einem Heißschmelzklebstoff angeklebt ist, welcher ferner in einer einheitlichen Mischung umfaßt:
etwa 10% bis etwa 50% eines Blockcopolymers, wobei das Blockcopolymer eine lineare oder radiale Copolymerstruktur mit der Formel (A-B)ₓ ist, wobei Block A ein Polyvinylarenblock ist, Block B ein Poly(monoalkenyl)block ist, x die Anzahl an polymeren Armen bezeichnet und wobei x eine ganze Zahl größer oder gleich Eins ist;
etwa 20% bis etwa 80% eines klebrig machenden Harzes; und
etwa 1 % bis etwa 60% eines wäßrigen, Flüssigkeit absorbierenden Polymers.

9. Absorbensgegenstand, der eine flüssigkeitspermeable Oberlage, eine flüssigkeitsimpermeable Barrierelage, ein Absorbenselement zwischen der Oberlage und der Barrierelage umfaßt, wobei wenigstens ein Bereich des Absorbenselements einen Heißschmelzklebstoff enthält, welcher weiter in einer einheitlichen Mischung umfaßt:
etwa 10% bis etwa 50% eines Blockcopolymers, wobei das Blockcopolymer eine lineare oder radiale Copolymerstruktur mit der Formel (A-B)ₓ ist, wobei Block A ein Polyvinylarenblock ist, Block B ein Poly(monoalkenyl)block ist, x die Anzahl an polymeren Armen bezeichnet und wobei x eine ganze Zahl größer oder gleich Eins ist;
etwa 20% bis 80% eines klebrig machenden Harzes; und
etwa 1% bis etwa 60% eines wäßrigen, Flüssigkeit absorbierenden Polymers.

## Revendications

1. Adhésif thermofusible qui est capable d'absorber des liquides aqueux qui comprend dans un mélange uniforme :
environ 10 % à environ 50 % d'un copolymère séquencé, le copolymère séquencé étant une structure de copolymère linéaire ou radial ayant la formule (A-B)ₓ dans laquelle le bloc A est un bloc polyvinylarène, le bloc B est bloc poly(monoalcényle), x désigne le nombre de bras polymères et dans laquelle x représente un nombre entier supérieur ou égal à un ;
environ 20 % à environ 80 % d'une résine donnant du collant ;
et environ 1 % à environ 60 % d'un polymère aqueux absorbant le liquide, dans lequel le polymère aqueux absorbant le liquide est choisi dans le groupe constitué d'hydrogels thermoplastiques et de compositions polymères thermoplastiques qui sont formées à partir d'un segment souple soluble dans l'eau et d'un ou plusieurs segments durs.

2. Adhésif thermofusible selon la revendication 1, , dans lequel les polyvinylarènes du bloc A sont choisis dans le groupe constitué de polystyrène, polyalphaméthylstyrène, polyvinyltoluène et des combinaisons de ceux-ci et dans lequel les blocs du poly(monoalcényle) du bloc B sont choisis dans le groupe constitué d'élastomères diéniques conjugués, d'élastomères hydrogénés et de combinaisons de ceux-ci.

3. Adhésif thermofusible selon la revendication 2, dans lequel les élastomères diéniques conjugués sont choisis dans le groupe constitué de polybutadiène et de polyisoprène et dans lequel les élastomères hydrogénés sont choisis dans le groupe constitué d'éthylène-butylène, d'éthylène propylène, de polyisobutylène et de combinaisons de ceux-ci.

4. Adhésif thermofusible selon la revendication 1, dans lequel l'adhésif thermofusible contient en outre des polymères thermoplastiques absorbants, des particules super absorbantes, des agents donnant du collant et des plastifiants.

5. Adhésif thermofusible selon la revendication 1, dans lequel les résines donnant du collant sont choisies dans le groupe constitué de résines naturelles, résines modifiées, esters de glycérol de résines naturelles, esters de glycérol de résines modifiées, esters de pentaérythritol de résines naturelles, esters de pentaérythritol de résines modifiées ; résines de polyterpène, copolymères de terpènes naturels, terpolymères de terpènes naturels, résines de terpène modifiées phénoliques et les dérivés hydrogénés de ceux-ci, résines de pétrole aliphatiques et les dérivés hydrogénés de celles-ci, résines de pétrole aromatiques et les dérivés hydrogénés de celles-ci, résines de pétrole aliphatiques, dérivés hydrogénés des résines de pétrole aliphatiques, résines de pétrole aromatiques, dérivés hydrogénés des résines de pétrole aromatiques et des combinaisons de ceux-ci.

6. Adhésif thermofusible selon l'une quelconque des revendications précédentes, dans lequel les segments durs sont choisis dans le groupe constitué de polyuréthane, polyamides, polyesters, polyurées, oxyde de polypropylène et des combinaisons de ceux-ci.

7. Adhésif thermofusible selon l'une quelconque des revendications précédentes, dans lequel les segments souples sont choisis dans le groupe constitué d'oxyde de polyéthylène, d'alcool polyvinylique, de pyrrolidone polyvinylique, de polyacrylamide, de polysaccharide, d'anhydride polymaléique et de copolymères statistiques d'oxyde de polyéthylène et d'oxyde de polypropylène.

8. Objet absorbant comprenant une couche supérieure perméable aux liquides, une couche barrière imperméable aux liquides, un élément absorbant entre la couche supérieure et la couche barrière, dans lequel soit la couche supérieure soit la couche barrière est mise à adhérer à l'élément absorbant avec un adhésif thermofusible qui comprend en outre dans un mélange uniforme:
environ 10 % à environ 50 % d'un copolymère séquencé, le copolymère séquencé étant une structure de copolymère linéaire ou radial ayant la formule (A-B)ₓ dans laquelle le bloc A est un bloc polyvinylarène, le bloc B est bloc poly(monoalcényle), x désigne le nombre de bras polymères et dans laquelle x représente un nombre entier supérieur ou égal à un ;
environ 20 % à environ 80 % d'une résine donnant du collant ; et
environ 1 % à environ 60 % d'un polymère aqueux absorbant le liquide.

9. Objet absorbant comprenant une couche supérieure perméable aux liquides, une couche barrière imperméable aux liquides, un élément absorbant entre la couche supérieure et la couche barrière, dans lequel au moins une partie de l'élément absorbant contient un adhésif thermofusible qui comprend en outre dans un mélange uniforme :
environ 10 % à environ 50 % d'un copolymère séquencé, le copolymère séquencé étant une structure de copolymère linéaire ou radial ayant la formule (A-B)ₓ dans laquelle le bloc A est un bloc polyvinylarène, le bloc B est bloc poly(monoalcényle), x désigne le nombre de bras polymères et dans laquelle x représente un nombre entier supérieur ou égal à un ;
environ 20 % à environ 80 % d'une résine donnant du collant ; et
environ 1 % à environ 60 % d'un polymère aqueux absorbant le liquide.
